# EUROPEAN PATENT APPLICATION

(11) **EP 2 287 145 A1**
(43) Date of publication of application: **23.02.2011**
(21) Application number: 09251984.2
(22) Date of filing: 12.08.2009
(51) Int. Cl.: C07C 7/00, C07C 11/04

(54) **Process for purifying ethylene**

(71) Applicant: BP p.l.c., London SW1Y 4PD (GB)
(72) Inventor: The designation of the inventor has not yet been filed
(74) Representative: Hill, Simon Stephen

(57) **Abstract**

The present invention relates to a process for purifying ethylene from a feedstock comprising ethylene and water.

More particularly, the present invention relates to a process for purifying ethylene from a feedstock comprising ethylene, diethyl ether and water.

## Description

The present invention relates to a process for purifying ethylene from a feedstock comprising ethylene and water.

More particularly, the present invention relates to a process for purifying ethylene from a feedstock comprising ethylene, diethyl ether and water.

Olefin(s) have traditionally been produced by steam or catalytic cracking of hydrocarbons. However, inevitably as oil resources decrease the price of oil will continue to increase; making light olefin(s) production a costly process. Thus there is an ever-growing need for non-petroleum routes to produce C₂+ olefin(s), essentially ethylene and propylene. Such olefin(s) are useful starting materials for numerous chemical products including polymeric products such as polyethylene and polypropylene.

In recent years the search for alternative materials for C2+ olefin(s) production has led to the use of alcohols such as methanol, ethanol and higher alcohols. The said alcohols may be produced by the fermentation of, for example, sugars and/or cellulosic materials.

Alternatively, alcohols may be produced from synthesis gas (also known as "syngas"). Synthesis gas refers to a combination of hydrogen and carbon oxides produced in a synthesis gas plant from a carbon source such as natural gas, petroleum liquids, biomass and carbonaceous materials including coal, recycled plastics, municipal wastes, or any organic material. Thus, alcohol and alcohol derivatives may provide non-petroleum based routes for the production of olefin(s) and other related hydrocarbons.

Processes for producing mixtures of carbon oxide(s) and hydrogen (synthesis gas) are well known. Each has its advantages and disadvantages and the choice of using a particular reforming process is dictated by economic and available feed stream considerations, as well as by the desired mole ratio of H2:CO in the feedstock resulting from the reforming reaction. The synthesis gas may be prepared using any of the processes known in the art including partial oxidation of hydrocarbons, steam reforming, gas heated reforming, microchannel reforming (as described in, for example, US 6,284,217 which is herein incorporated by reference), plasma reforming, autothermal reforming and any combination thereof. A discussion of these synthesis gas production technologies is provided in "Hydrocarbon Processing" V78, N.4, 87-90, 92-93 (April 1999) and "Petrole et Techniques", N. 415, 86-93 (July-August 1998). It is also known that the synthesis gas may be obtained by catalytic partial oxidation of hydrocarbons in a microstructured reactor as exemplified in "IMRET 3: Proceedings of the Third International Conference on Microreaction Technology", Editor W Ehrfeld, Springer Verlag, 1999, pages 187-196. Alternatively, the synthesis gas may be obtained by short contact time catalytic partial oxidation of hydrocarbonaceous feedstocks as described in EP 0303438. Typically synthesis gas is obtained via a "Compact Reformer" process as described in "Hydrocarbon Engineering", 2000, 5, (5), 67-69; "Hydrocarbon Processing", 79/9, 34 (September 2000); "Today's Refinery", 15/8, 9 (August 2000); WO 99/02254; and WO 200023689.

Typically, for commercial syngas production the pressure at which the synthesis gas is produced ranges from approximately 20 to 75 bar and the temperature at which the synthesis gas exits the reformer ranges from approximately 700 DEG C. to 1100 DEG C. The synthesis gas contains a molar ratio of hydrogen to carbon oxide - which is dependent on the syngas feedstock - ranging from 0.8 to 3.

The Applicants have developed several routes for producing an oxygenate alcohols from syngas, inter alia EP 1888492, EP1904426, EP2024315, EP2021310, EP 1914219, EP1923380, WO2008/047103, WO2009/063176, EP2060553, EP2060555, WO2009/077723, WO2009/077719, WO2009/077729, WO2009/077730, WO2009/077720, WO2009/077726, EP2060555, EP2072488, WO2009/063173, W02009/063174.

The Applicants have also developed several routes for converting an oxygenate feedstock comprising alcohols into liquid hydrocarbons comprising C2 -C4 olefin(s), inter alia EP 1899284, EP 1904423, EP 1902006, EP 1954654, EP 1954655, EP 1954656, EP1954657, EP2089156, EP1925363, WO2009/050433, WO2009/074774.

Before use in downstream industrial applications, e.g. ethylene polymerization, the ethylene feed must undergo a very important purification procedure. For example, only ppm of water content in the olefin feed are acceptable by the polymer industry because said water would act as a catalyst poison. There are also other reasons why water should be removed from the olefin stream: indeed, during the conditioning of ethylene, the downstream equipment is usually operated at very low temperatures, e.g. -28 degC, i.e. temperatures at which any water present may freeze, giving important operational difficulties.

Different methods have been described in the prior art in order to remove the water from ethylene. The use of molecular sieves is the most common method used and it has been extensively described in the literature over the years. The molecular sieve method however appears to be problematic in a number of ways for the following reasons:
i) It is a semi-batch operation,
ii) Ethylene may polymerise on the molecular sieve material,
iii) It is very difficult to control the method to achieve the desired low levels of water content (e.g. 1-2 ppm),
iv) Other organic components may be simultaneously caught on the molecular sieve material, and be either lost in regeneration or need expensive additional recovery methods, and
v) The pressure drop created by the molecular sieve separation method will increase the size of the ethylene downstream compressor.

In the course of their continuous efforts of developments for improving their ethylene production technology, the Applicants have unexpectedly found a very simple, elegant and advantageous method which allows to separate efficiently the water present in the olefin stream whilst avoiding the problems encountered by the prior art methods.

In one of their most preferred ethylene production technologies from the dehydration of an alcohol feedstock, the crude ethylene stream obtained after dehydration of the alcohol feedstock comprises essentially ethylene, diethyl ether and water. The new method developed by the Applicants consists in using advantageously the diethyl ether present in the crude ethylene stream to separate the water from said crude ethylene stream. This new method and all the advantages associated therewith will be disclosed hereafter in more details.

Thus, the present invention provides for a continuous process for removing water from a crude ethylene stream **characterized in that**
- said crude ethylene stream is introduced into and circulated through a separation vessel,
- a liquid diethyl ether is introduced into and circulated through the said separation vessel in a countercurrent flow with the said crude ethylene stream, and
- a purified ethylene stream is recovered.

For the purpose of the present invention and appended claims, this continuous process will be designated as the scrubber process, the diethyl ether is the scrubbing feed, water is the scrubbed feed and the separation vessel is the scrubber; the purified ethylene stream is the crude ethylene stream from which a substantial amount of water has been removed by the scrubber process according to the present invention.

According to a preferred embodiment of the present invention, before introduction in the scrubber, the crude ethylene stream, which is preferably in a gaseous state, comprises at least 60wt% ethylene, preferably at least 70wt% ethylene, more preferably at least 75wt% ethylene.

According to a preferred embodiment of the present invention, before introduction in the scrubber, the crude ethylene stream comprises enough diethyl ether for scrubbing the water present in the stream, i.e. preferably at least 2wt% of diethyl ether, more preferably at least 10wt% of diethyl ether, more preferably at least 15wt% of diethyl ether. Indeed, according to a preferred embodiment of the present invention, the scrubbing feed is already present in the crude ethylene stream to be purified from water, which makes the whole scrubber operations very efficient as explained hereafter.

According to the present invention, before introduction in the scrubber, the crude ethylene stream comprises water, preferably in an amount comprised between 0.05 and 0.5 wt%.

According to a preferred embodiment of the present invention, before introduction in the scrubber, the total amount of ethylene, diethyl ether and water in the crude ethylene stream is of at least 95wt%, preferably at least 99wt%, more preferably 100wt%.

Other oxygenates may also be tolerated in small amounts in the crude ethylene stream; for example acetaldehyde and/or ethanol may also be present, e.g. at a content of preferably less than 0.5wt%.

According to the present invention, the temperature and pressure conditions of the crude ethylene stream before introduction in the scrubber are such that the water does not freeze. Preferably, said conditions are such that the temperature is at least 5 degrees C higher than the greater of the liquid freezing point and vapour reverse sublimation point at the respective pressure and composition.

The preferred pressure of the crude ethylene stream before its introduction in the scrubber is at least 0.5MPa, more preferably at least 1MPa, most preferably at least 1.5MPa. A pressure in the range of 1.5MPa to 3MPa is preferably used.

The preferred temperature of the crude ethylene stream before its introduction in the scrubber is at least 0degreeC, more preferably at least 10degrees, most preferably at least 15degreesC. A temperature in the range of 20 to 50degreesC is preferably used.

According to the present invention, the scrubber process can be performed in any appropriate separation vessel. For example, the separation may be effected in any device designed for contact between vapour and liquid phases and phase separation. Such separation devices may be single or multi-staged, and include, but are not limited to: flash vessels; or distillation vessels, employing sieve trays; valve trays; structured packing or random packing.

Reflux may be provided to the separation vessel by partial condensation of the overhead vapour. Such condensation may take place in any suitable heat transfer device, including, but not limited to the following types of heat exchanger: shell and tube, including any means of extending the heat transfer surface of the tube; double tube; fin/fan; plate and frame compact of gasketted or welded manufacture; printed circuit; spiral-wound, falling film or spinning disc.

Reflux may be augmented or replaced with a liquid stream containing the scrubbing hydrocarbon sourced from further separation of the gas or liquid effluent from the separator. Such a stream may also or alternatively be introduced elsewhere in the column, and may be introduced in the liquid and/or vapour phase.

Cooling in the condenser may optionally be effected by indirect heat transfer with a coolant, examples of which include, but are not limited to: ethylene and/or propylene glycol solution; propylene; ethylene; proprietary organic silica fluids. Optionally, cooling in the condenser may be effected by direct heat transfer, for example with olefin.

According to a preferred embodiment of the present invention, the scrubber is a multi-staged distillation column. The crude ethylene feed is introduced in the vapour phase, the stream including olefin(s), the scrubbing oxygenate; trace water; and other materials. Reflux is provided to the column by partial condensation of the overhead vapour. The partial condenser is cooled through indirect heat transfer. Overhead vapour leaves the partial condenser including ethylene, scrubbing oxygenate and is marked by a reduced content of water when compared to the crude olefin feed. A liquid stream is removed from the distillation column, comprising scrubbing oxygenate and water.

Whilst not wishing to be bound by this theory, the Applicants also believe that it is preferred to conduct the separation in a multi-stage distillation column with trays rather than packing, as low temperature operation may give rise to liquid with surface tension high enough to prevent effective use of structured or random packing.

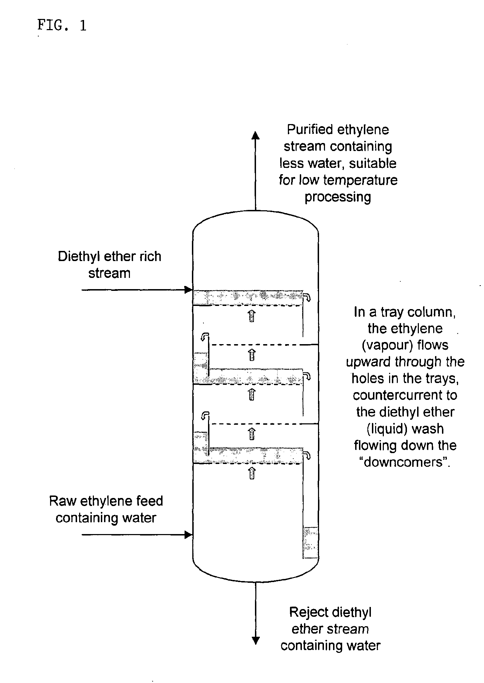

The invention will also be illustrated with the appended figure which is not intended to restrict the scope of the invention to its specific embodiment.

## Claims

1. Continuous process for removing water from a crude ethylene stream **characterized in that**
- said crude ethylene stream is introduced into and circulated through a separation vessel,
- a liquid diethyl ether is introduced into and circulated through the said separation vessel in a countercurrent flow with the said crude ethylene stream, and
- a purified ethylene stream is recovered.

2. Process according to claim 1 wherein, before introduction in the scrubber, the crude ethylene stream, which is preferably in a gaseous state, comprises at least 60wt% ethylene, preferably at least 70wt% ethylene, more preferably at least 75wt% ethylene.

3. Process according to any of the preceding claims wherein, before introduction in the scrubber, the crude ethylene stream comprises enough diethyl ether for scrubbing the water present in the stream, i.e. preferably at least 2wt% of diethyl ether, more preferably at least 10wt% of diethyl ether, more preferably at least 15wt% of diethyl ether.

4. Process according to any of the preceding claims wherein, before introduction in the scrubber, the crude ethylene stream comprises water in an amount comprised between 0.05 and 0.5 wt%.

5. Process according to any of the preceding claims wherein, before introduction in the scrubber, the total amount of ethylene, diethyl ether and water in the crude ethylene stream is of at least 95wt%, preferably at least 99wt%, more preferably 100wt%.

6. Process according to any of the preceding claims wherein the temperature and pressure conditions of the crude ethylene stream before introduction in the scrubber are such that the temperature is kept such that the temperature is at least 5 degrees C higher than the greater of the liquid freezing point and vapour reverse sublimation point at the respective pressure and composition.

7. Process according to any of the preceding claims wherein the pressure of the crude ethylene stream before its introduction in the scrubber is at least 0.5MPa, more preferably at least 1MPa, most preferably at least 1.5MPa.

8. Process according to claim 7 wherein a pressure is in the range of 1.5MPa to 3MPa.

9. Process according to any of the preceding claims wherein the temperature of the crude ethylene stream before its introduction in the scrubber is at least 0degreeC, more preferably at least 10degreesC, most preferably at least 15degrees.

10. Process according to claim 9 wherein the temperature is in the range of 20 to 50degreesC.
